# EUROPEAN PATENT APPLICATION

(11) **EP 2 719 703 A1**
(43) Date of publication of application: **16.04.2014**
(21) Application number: 12188411.8
(22) Date of filing: 12.10.2012
(51) Int. Cl.: C07K 1/13

(54) **Precursor molecule for the synthesis of D-luciferin**

(71) Applicant: Ecole Polytechnique Fédérale de Lausanne (EPFL), 1015 Lausanne (CH)
(72) Inventor: Dubikovskaya, Elena, 1025 St. Sulpice (CH); Godinat, Aurelien, 1024 Ecublens (CH)
(74) Representative: Hepp Wenger Ryffel AG

(57) **Abstract**

The present invention relates to precursor molecules for the synthesis of D-luciferin, or blocked D-luciferin, which are functionalised in different positions. Further, the use of functionalised precursor molecules for the synthesis of D-luciferin or blocked D-luciferin and methods of synthesising blocked D-luciferin and D-luciferin are described. Also described is a kit of parts for screening assay comprising said functionalised precursor molecules to D-luciferin or blocked D-luciferin and the uses of such a screening assay for the detection of molecules for the study of molecular uptake and for the detection of a reducing environment. Methods of detection bio- molecules and for determining a sequence within a peptide of protein cleaved by specific enzymes are described. Also a method of determining the cysteine concentration in a cell or tissue sample is described. Precursor molecules of D-luciferin according to the invention are advantageous as they are small and travel easily around live systems and allow tissue cells or organs to be targeted specifically. Further, the methods and screening assays described allow the simultaneous study of multiple biological processes in one experiment only.

## Description

The present invention relates to functionalized precursor molecules for the synthesis of D-luciferin, amino-D-luciferin, blocked D-luciferin or blocked amino-D-luciferin. Further, the use of functionalized precursor molecules for the synthesis of D-luciferin, amino-D-luciferin, blocked D-luciferin or blocked amino-D-luciferin and methods of synthesising D-luciferin, amino-D-luciferin, blocked D-luciferin and blocked amino-D-luciferin are described. Also described is a kit of parts for a screening assay comprising said functionalised precursor molecules to D-luciferin, amino-D-luciferin, blocked D-luciferin or blocked amino-D-luciferin and the uses of such a screening assay for the detection of molecules, for the study of cellular uptake of molecules and for the detection of a reducing hypoxic environment and for the determination of peptide or protein sequences cleaved by a specific enzyme. Also a method of determining the cysteine concentration in a cell or tissue sample is described.

D-luciferin is a molecule that is oxidized by the enzyme luciferase in the mandatory presence of adenosine triphosphate (ATP), oxygen and a magnesium cation source, giving rise to luminescence in this reaction. Amino-D-luciferin also undergoes the same reaction. Thus the presence of oxygen, magnesium cations, ATP, luciferase and D-luciferin or amino-D-luciferin is indicated by a luminescent signal. If the functional groups of D-luciferin or amino-D-luciferin are blocked by reactions with other molecules, D-luciferin's or amino-D-luciferin's oxidation cannot be catalyzed by the enzyme luciferase, i.e. no luminescent signal occurs.

By functionalized D-luciferin or amino-D-luciferin herein and below the derivatives of both D-luciferin and amino-D-luciferin functionalized at any possible position of the molecule should be understood. By functionalized 6-hydroxy-2-cyanobenzothiazole or 6-amino-2-cyanobenzothiazole herein and below the derivatives of 6-hydroxy-2-cyanobenzothiazole or 6-amino-2-cyanobenzothiazole functionalized at any possible position of the molecule should be understood.

Known methods of utilizing the reaction between luciferase and D-luciferin or amino-D-luciferin involve the synthesis of blocked D-luciferin ot blocked amino-D-luciferin prior to addition to the system to be studied. In terms of the usage of blocked D-luciferin or blocked amino-D-luciferin in the prior art, usually only one biological process, i.e. the activity of one biomolecule, is studied at a time.

Investigation of such a process occurs by reaction of the molecule studied with the help of the blocked D-luciferin or blocked amino-D-luciferin in such a way that the group or the groups blocking the OH, NH₂ or carboxyl functionalities within blocked D-luciferin or blocked amino-D-luciferin is removed to yield unfunctionalized D-luciferin or amino-D-luciferin. Unfunctionalized D-luciferin or amino-D-luciferin can then undergo a reaction with luciferase. The luminescent signal observed indicates that the molecule under investigation is in fact present in the system studied. Said molecule or a biological process associated to it removes the groups blocking the functional groups of D-luciferin or amino-D-luciferin, which originally prevented a reaction between D-luciferin or amino-D-luciferin and luciferase.

It is the object of the present invention to overcome the drawbacks of the prior art. In particular the present invention aims to provide small precursor molecules that travel easily around live systems due to their small size, allow certain tissue cells or organs to be targeted specifically and only react to form D-luciferin, amino-D-luciferin, blocked D-luciferin, blocked amino-D-luciferin or other conjugates with biological or radioactive or fluorescent reporting activity once they have been added to the cell or tissue sample or organism to be studied. Another object is the simultaneous study of two or more biological processes in one experiment only.

It has surprisingly been found that this object is achieved by the subject matter of present claim 1. Claim 1 describes a precursor molecule for the synthesis of D-luciferin or amino-D-luciferin or blocked D-luciferin or blocked amino-D-luciferin, chosen from the group consisting of
a) functionalized D-cysteine, wherein the functionalization does not prevent the reactivity towards 6-hydroxy-2-cyanobenzothiazole or 6-amino-2-cyanobenzothiazole in vitro and in vivo; and wherein when reacting with a molecule according to b) or when reacting within unfunctionalized 6-hydroxy-2-cyanobenzothiazole or 6-amino-2-cyanobenzothiazole in vitro and in vivo a fuctionalized D-luciferin or a functionalized amino-D-luciferin molecule is obtained, which is not readily reactive towards luciferase;
b) functionalized 6-hydroxy-2-cyanobenzothiazole or 6-amino-2-cyanobenzothiazole, wherein the functionalization does not prevent the reactivity in vitro and in vivo towards D-cysteine or other functionalized or unfunctionalized aminothiols, or functionalized or unfunctionalized 2-aminobenzylamines; and wherein, when reacting with a molecule according to a) or when reacting within unfunctionalized D-cysteine, a functionalized D-luciferin or a functionalized amino-D-luciferin molecule is obtained, which is not readily reactive towards luciferase;
c) functionalized D-cysteine, wherein the functionalization prevents the reactivity towards 6-hydroxy-2-cyanobenzothiazole or 6-amino-2-cyanobenzothiazole in vitro and in vivo;
d) functionalized 6-hydroxy-2-cyanobenzothiazole or 6-amino-2-cyanobenzothiazole, wherein the functionalization prevents the reactivity towards D-cysteine, or other functionalized or unfunctionalized aminothiols or functionalized or unfunctionalized 2-aminobenzylamines.

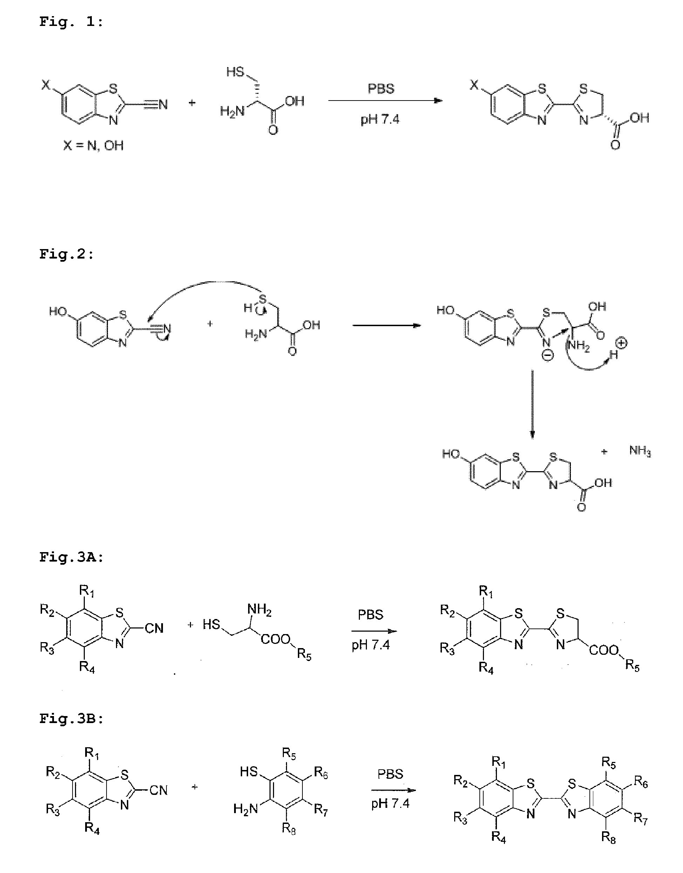

A common synthesis route of unfunctionalized D-luciferin or amino-D-luciferin, which is oxidized by the enzyme luciferase in the presence of ATP, oxygen and magnesium cations to yield light in a bioluminescent reaction, involves as precursors an unfunctionalized 2-cyano-6-hydroxybenzothiazole (OH-CBT) or an unfunctionalized 2-cyano-6-aminobenzothiazole (NH₂-CBT) and an unfunctionalized D-cysteine (D-Cys) as shown in Fig. 1 and 2, hereinbelow.

Instead of synthesizing D-luciferin or amino-D-luciferin using the unfunctionalized precursors depicted in Fig. 1 and 2 hereinbelow, there are a number of conceivable other synthesis routes that can be used to prepare functionalized or unfunctionalized D-luciferin or amino-D-luciferin molecules in vivo or in vitro using several combinations of functionalized and unfunctionalized precursor molecules, i.e. functionalized or unfunctionalized D-cysteine and functionalized or unfunctionalized 6-hydroxy-2-cyanobenzothiazole or 6-amino-2-cyanobenzothiazole or functionalized or unfunctionalized aminothiols other than D-cysteine or functionalized or unfunctionalized 2-aminobenzylamines, examples of which are depicted in Fig.3A to C and Fig. 4A and B.

While the term functionalization with regard to the D-luciferin or amino-D-luciferin molecule means that the molecule can be functionalized at any possible position available within the molecule, it particularly means that the molecule does not possess a free NH₂- or OH- group in the 6-position of its phenyl ring and/or no free carboxyl-group on the 5-membered ring of the molecule that is derived from D-cysteine. Rather, these groups are blocked or masked, i.e. rendered unreactive, by reacting with compounds such as chloroformates, carboxylic acids, organic acid anhydrides, alkylating agents for hydroxyl- or amino groups of luciferin and alcohols and amines in case of carboxylic groups of luciferin to yield groups such as esters, ethers and amides. Alternatively, blocking of these groups can be achieved by adding onto them a sequence of amino acids which may be part of a peptide or protein. Such blocking or masking is reversible under certain conditions, for instance when biomolecules such as specific enzymes are present that are capable of removing the functionalities that block the groups attached to D-luciferin or amino-D-luciferin. A D-luciferin or amino-D-luciferin molecule functionalized in this way is termed "blocked" or "caged" or "functionalized" D-luciferin or amino-D-luciferin. These terms can be used interchangeably. A blocked D-luciferin or amino-D-luciferin cannot readily react with the enzyme luciferase to undergo a bioluminescent reaction by oxidation that emits light, which can then be detected and measured.

The precursors for the synthesis of blocked derivatives of D-luciferin or amino-D-luciferin according to the invention are: D-cysteine possessing a blocked carboxyl-group, D-cysteine possessing a blocked carboxyl-group as well as a blocked thiol group and/or as well as a blocked amino-group, 6-hydroxy-2-cyanobenzothiazole or 6-amino-2-cyanobenzothiazole possessing a blocked amino or hydroxyl-group, 6-hydroxy-2-cyanobenzothiazole or 6-amino-2-cyanobenzothiazole possessing a blocked amino or hydroxyl-group as well as a blocked nitrile-group. In order to obtain a blocked D-luciferin or a blocked amino-D-luciferin molecule, the specific D-cysteine and cyanobenzothiazole molecules listed above may or may not be used with each other in the same reaction.

Alternatively, either one of the blocked D-cysteine molecules listed or the blocked 6-hydroxy-2-cyanobenzothiazole or 6-amino-2-cyanobenzothiazole molecules listed can be used and added to the respective unfunctionalized 6-hydroxy-2-cyanobenzothiazole or 6-amino-2-cyanobenzothiazole or unfunctionalized D-cysteine molecule.

The precursors for the synthesis of D-luciferin or amino-D-luciferin according to the invention are: D-cysteine possessing a blocked thiol-group and/or a blocked amino-group and 6-hydroxy-2-cyanobenzothiazole or 6-amino-2-cyanobenzothiazole possessing a blocked nitrile-group. In order to obtain a D-luciferin or an amino-D-luciferin molecule, the specific D-cysteine and 6-hydroxy-2-cyanobenzothiazole or 6-amino-2-cyanobenzothiazole molecules listed above may or may not be used with each other in the same reaction.

Alternatively, a blocked D-cysteine described may be added to an unfunctionalized 6-hydroxy-2-cyanobenzothiazole or 6-amino-2-cyanobenzothiazole molecule or a blocked 6-hydroxy-2-cyanobenzothiazole or 6-amino-2-cyanobenzothiazole may be added to an unfunctionalized D-cysteine molecule.

Also encompassed by the present invention is the use of a functionalized D-cysteine according to compounds a) or c) as described above or a functionalized 6-hydroxy-2-cyanobenzothiazole or 6-amino-2-cyanobenzothiazole according to compounds b) or d) as described above for the synthesis of D-luciferin, amino-D-luciferin, a blocked D-luciferin or a blocked amino-D-luciferin.

Another aspect of the invention is the method of synthesizing blocked D-luciferin or blocked amino-D-luciferin, wherein
i) a functionalized D-cysteine according to a) as described for the precursors above is mixed and allowed to react with either a functionalized 6-hydroxy-2-cyanobenzothiazole or 6-amino-2-cyanobenzothiazole according to b) as described for the precursors above; or with unfunctionalized 6-hydroxy-2-cyanobenzothiazole or 6-amino-2-cyanobenzothiazole;
   or, wherein
ii) a functionalized 6-hydroxy-2-cyanobenzothiazole or 6-amino-2-cyanobenzothiazole according to b) as described for the precursors above is mixed and allowed to react with either a functionalized D-cysteine according to a) as described for the precursors above; or with unfunctionalized D-cysteine.

The functionalized D-cysteine of method step i) is a D-cysteine whose carboxyl function is blocked and which is not prevented from reacting with a 6-hydroxy-2-cyanobenzothiazole or 6-amino-2-cyanobenzothiazole molecule via its amino and thiol-groups. The functionalized 6-hydroxy-2-cyanobenzothiazole or 6-amino-2-cyanobenzothiazole of method step ii. is a 6-hydroxy-2-cyanobenzothiazole or 6-amino-2-cyanobenzothiazole whose OH or NH₂ group is blocked and which is not prevented from reacting with D-cysteine via its nitrile group.

In order to obtain blocked D-luciferin or blocked amino-D-luciferin, the functionalized D-cysteine of route i) can be added to either a functionalized 6-hydroxy-2-cyanobenzothiazole or 6-amino-2-cyanobenzothiazole or to an unfunctionalized 6-hydroxy-2-cyanobenzothiazole or 6-amino-2-cyanobenzothiazole. Blocked D-luciferin or blocked amino_D-luciferin is also synthesised when a blocked 6-hydroxy-2-cyanobenzothiazole or 6-amino-2-cyanobenzothiazole of route ii) is added to either a functionalized D-cysteine or to an unfunctionalized D-cysteine.

The invention further encompasses a method of synthesizing functionalized D-luciferin or functionalized amino-D-luciferin in vitro and in vivo comprising the steps of
i) providing either a functionalized D-cysteine according to compounds a) or c) as described for the precursors above or a functionalized 6-hydroxy-2-cyanobenzothiazole or 6-amino-2-cyanobenzothiazole according to compounds b) or d) as described for the precursors above,
ii) providing the respective complementary unfunctionalized or functionalized 6-hydroxy-2-cyanobenzothiazole or 6-amino-2-cyanobenzothiazole or unfunctionalized or functionalized D-cysteine,
iii) adding the components provided in steps i) and ii) to a system containing one or more biomolecules capable of cleaving functional groups off functionalized D-cysteine and /or functionalized 6-hydroxy-2-cyanobenzothiazole or 6-amino-2-cyanobenzothiazole and allowing them to react.

A functionalized D-cysteine of method step i) is a D-cysteine molecule that is blocked at its carboxylic group and/or its SH-group and/or its NH₂-group.

A functionalized 6-hydroxy-2-cyanobenzothiazole or 6-amino-2-cyanobenzothiazole according to method step ii) is a cyanobenzothiazole, which is blocked at its NH₂ or OH-group and/or at its nitrile group.

Both the blocked functional groups of the functionalized D-cysteine and the functionalized 6-hydroxy-2-cyanobenzothiazole or functionalized 6-amino-2-cyanobenzothiazole molecules according to steps i) and ii) must be unblocked prior to formation of blocked D-luciferin or blocked amino-D-luciferin or after such formation in order to yield free D-luciferin or free amino-D-luciferin by one or more suitable molecules, preferably biomolecules, more preferably by enzymes, for subsequent reaction with luciferase and detection of the emitted light.

When the reactions between 6-hydroxy-2-cyanobenzothiazole or 6-amino-2-cyanobenzothiazole and D-cysteine in vivo or in vitro involve unblocking of the precursors prior to a reaction yielding D-luciferin or amino-D-luciferin, the formation of D-luciferin or amino-D-luciferin takes place only gradually. Currently used approaches in vivo use injectinon of already synthesized D-luciferin or amino-D-luciferin, which provides a rapid boost of light emission with the subsequent rapid decay of signal due to the enzyme luciferase inhibition. Alternatively, a drug pump is surgically implanted containing free D-luciferin or free amino-D-luciferin. This procedure is stressful for the animal. Injecting separately blocked D-cysteine and blocked 6-hydroxy-2-cyanobenzothiazole or blocked 6-amino-2-cyanobenzothiazole will provide gradual formation of D-luciferin or amino-D-luciferin over time, thus overcoming the drawback of luciferase inhibition and avoiding the exposure of the animal to a stressful situation.

Another aspect of the invention is a kit of parts for a screening assay for the detection of molecules in vivo or in vitro, which comprises
- either a functionalized D-cysteine according to compounds a) or c) as described for the precursors above or a functionalized 6-hydroxy-2-cyanobenzothiazole or 6-amino-2-cyanobenzothiazole according to compounds b) or d) as described for the precursors above,
- the respective complementary unfunctionalized or functionalized 6-hydroxy-2-cyanobenzothiazole or 6-amino-2-cyanobenzothiazole or unfunctionalized or functionalized D-cysteine,
- optionally, luciferase,
- optionally, ATP,
- optionally, a magnesium cation source, and
- instructions for use.

This kit of parts is characterized in that it contains separately the above mentioned component of a functionalized D-cysteine according to compounds a) or c) as described for the precursors above or a functionalized 6-hydroxy-2-cyanobenzothiazole or a functionalized 6-amino-2-cyanobenzothiazole according to compounds b) or d) from the second component of a respective complementary functionalized or unfunctionalized 6-hydroxy-2-cyanobenzothiazole or 6-amino-2-cyanobenzothiazole or unfunctionalized or functionalized D-cysteine. Further, if comprised within the kit, luciferase is also kept separately from the previous two kit components. Luciferase is an optional part of the kit, i.e. it may be contained within the kit or it may be expressed by the cells of a sample to be studied.Also, optionally comprised within the kit and kept separately from other kit components and each other are adenosine triphosphate (ATP) and a magnesium cation source, which may be a magnesium cation solution. The kit also comprises instructions for use explaining how the individual kit components are to be used; moreover, such instruction for use may also prompt the user to obtain further material (or material indicated as "optional" herein) from other sources and/or separately. This definition of "instructions for use" applies wherever it is used herein.

Molecules to be screened by the screening assay kit of parts are preferably biomolecules, more preferably enzymes, which react with blocked groups of the precursors and/or the blocked groups of functionalized D-luciferin or functionalized amino-D-luciferin thereby releasing the respective free precursors or free D-luciferin or free amino-D-luciferin. Typical enzymes are hydrolases such as proteases and peptidases, more specifically caspase, thrombin, trypsin, SARS-protease, cathepsins, kallik-rein, aminopeptidase, prostate specific antigen, dipeptidyl peptidase, caplain, beta-galactosidase and phosphatase. Other molecules that could be detected in this way by the screening assay are azides and hydrogen peroxides.

Yet another aspect of the invention are the uses of a kit of parts for a screening assay as described above.

Said kit of parts can be used for the detection of molecules in vivo or in vitro. Detection of one type of molecule can be achieved by adding to the cell or tissue sample or organism to be investigated one precursor blocked in one position, followed by the unfunctionalized corresponding precursor that is needed to synthesize D-luciferin or amino-D-luciferin. Optionally, i.e. if necessary, luciferase and/or a magnesium cation source and/or ATP are added to the sample studied. If the molecule necessary to remove the blocking is present, D-luciferin or amino-D-luciferin will form upon removal of the blocking and then react with luciferase to give rise to luminescence. The luminescent signal may vary in intensity depending on the concentrations of the molecule investigated and depending on those of the different kit components.

It is also possible to investigate the presence of several molecules at the same time within the same sample. This is done by adding D-luciferin precursors or amino-D-luciferin precursors to the sample under investigation, which may be blocked in any number of available positions, i.e. in any of five different positions in total. These can then be unblocked by the molecules present and undergo the reaction to form D-luciferin or amino-D-luciferin, followed by the reaction with the enzyme luciferase.

Alternatively, the presence of two or more different molecules at the same time within a cell or tissue sample or the occurrence of two or more biological processes such as reactions at the same time within a cell or tissue sample can be studied by employing the reactions to form functionalized or unfunctionalized D-luciferin or functionalized or unfunctionalized amino-D-luciferin as bio-orthogonal reactions alongside the well-known Staudinger reaction in vitro or in vivo. The term bio-orthogonal reaction describes the reaction between two reagents, which can only react with each other in living systems or cell or tissue samples but not with any other components found in the living system or cell or tissue sample, i.e. the reagents of a bio-orthogonal reaction do not interfere with the reactions and biological processes occurring in the system that is being studied. Further, the reagents that participate in one bio-orthogonal reaction do not interact with the reagents belonging to another bio-orthogonal reaction, such as the Staudinger reaction, that occurs simultaneously in a living system or cell or tissue sample.

A kit of parts for a screening assay as described above can further be used for the study of cellular uptake of molecules in vitro or in vivo. For instance, the uptake into a cell, both through endocytosis and receptor-mediated pathways, of a protein having a cysteine side-chain can be shown by first binding a D-cysteine to said protein thus forming a disulfide bond. Said protein is then added to cells pre-treated with 6-hydroxy-2-cyanobenzothiazole or 6-amino-2-cyanobenzothiazole. The reductive environment of the cell's cytosol containing glutathione will reduce the disulfide bond which will release a free D-cysteine. Free D-cysteine and free 6-hydroxy-2-cyanobenzothiazole or 6-amino-2-cyanobenzothiazole will then react to yield D-luciferin or amino-D-luciferin. If the cells investigated express luciferase or if luciferase is also added to the cell or tissue sample under investigation, D-luciferin or amino-D-luciferin and luciferase will react and give rise to luminescence, which can be detected and measured.

It is also conceivable that the uptake of other proteins having other kind of bonds cleaved by other cytosolic enzymes could be studied in this way.

Further, the use of a kit of parts for a screening assay as described above for the detection of a hypoxic environment in vivo or in vitro is encompassed by the present invention. In particular the presence of hypoxic reducing environments in cells, for instance caused by hypoxic tumours, can be studied. For instance, disulfide bonds or azo-compounds are known to be reduced into respectively thiols and amines in hypoxic environments. A D-cysteine blocked in both or either of its thiol- or amino-positions by hypoxia-induced removable groups can be added to cell or tissue samples to investigate hypoxia. If hypoxic conditions are present, both will be unblocked and can then react with unblocked 6-hydroxy-2-cyanobenzothiazole or 6-amino-2-cyanobenzothiazole also added to sample. If necessary, luciferase and/or a magnesium cation source and/or ATP is also added. If a hypoxic environment is present, D-cysteine and 6-hydroxy-2-cyanobenzothiazole or 6-amino-2-cyanobenzothiazole will react to yield D-luciferin or amino-D-luciferin, which will then react with luciferase to give rise to luminescence.

Yet another aspect of the invention is the method of detecting biomolecules comprising the steps of
i) providing either a functionalized D-cysteine according to compounds a) or c) as described for the precursors above or a functionalized 6-hydroxy-2-cyanobenzothiazole or 6-amino-2-cyanobenzothiazole according to compounds b) or d) as described for the precursors above,
ii) providing the respective complementary unfunctionalized or functionalized D-cysteine or unfunctionalized or functionalized 6-hydroxy-2-cyanobenzothiazole or 6-amino-2-cyanobenzothiazole compound,
iii) providing luciferase,
iv) contacting the components provided in steps i. and ii. and allowing them to react in the presence of one or more biomolecules studied, and
v) detecting and measuring the luminescence signal generated.

The above described method can be carried out in vivo or in vitro. Luciferase is either present in the sample investigated through cellular expression of this enzyme or will be added to the sample. Luminescent signals are detected and measured in accordance with routine procedures and equipment generally known in the art.

Further, a kit of parts for a screening assay for the determination of a sequence cleaved by a specific enzyme within a peptide or protein comprising
i) the peptide or protein bearing at the C-terminal end the sequence cleaved by the specific enzyme followed by a D-cysteine residue,
ii) providing an unfunctionalized 6-hydroxy-2-cyanobenzothiazole or an unfunctionalized 6-amino-2-cyanobenzothiazole,
iii) optionally, an enzyme capable of cleaving a sequence within the peptide or protein,
iv) optionally, luciferase,
v) optionally, magnesium cation source,
vi) optionally, ATP
vii) optionally, a means of detecting luminescence and measuring its intensity, and
viii) instructions for use is comprised by the present invention.

This kit of parts is characterized in that it contains separately the above mentioned component of a peptide or protein bearing at the C-terminal end the sequence cleaved by the specific enzyme followed by a D-cysteine residue, which could react with the second kit component, i.e. a free 6-hydroxy-2-cyanobenzothiazole or a free 6-amino-2-cyanobenzothiazole. Optionally and also separately, the kit may contain an enzyme capable of cleaving a sequence within the peptide or protein and a means of detecting luminescence and measuring its intensity. Also optionally and separately, luciferase may be contained within the kit. Alternatively, it is possible that luciferase is expressed by cells within a sample to be studied. Optionallyd separately, ATP and/or a magnesium cation source may be comprised within the kit. Another component of the kit of parts are instructions for use explaining in what sequence the individual kit components are to be added to tissue or cell samples or organisms.

Another aspect of the invention is the use of a kit of parts for a screening assay for the determination of a sequence within a peptide or protein cleaved by a specific enzyme as described above, whereby the individual kit components are utilized as is described for the method below. By using the kit of parts according to the invention, the best performing peptide sequence for a specific protease can be determined. Peptides with the desired sequence bearing at the C-terminal a D-cysteine after the cleavage site can be synthesized. Upon protease activity, the cleavage of the peptide will release a free D-cysteine, allowing the formation of D-luciferin or amino-D-luciferin if 6-hydroxy-2-cyanobenzothiazole or 6-amino-2-cyanobenzothiazole is present in solution. Luciferase will have to be present in the cells investigated or may optionally be added. The resulting reaction between D-luciferin or amino-D-luciferin and luciferase will give rise to luminescence. Intensity of light output will be proportional to the efficiency of peptide cleavage, allowing the determination of the best specific peptide sequence substrate for a particular enzyme.

Also, the method of determining a sequence within a peptide or protein cleaved by a specific enzyme comprising
i) providing a peptide or protein bearing at the C-terminal end the sequence cleaved by the specific enzyme followed by a D-cysteine residue,
ii) providing an unfunctionalized 6-hydroxy-2-cyanobenzothiazole or an unfunctionalized 6-amino-2-cyanobenzothiazole,
iii) providing an enzyme capable of cleaving a sequence within the peptide or protein,
iv) mixing the compounds of steps i., ii. and iii. and allowing them to react,
v) providing luciferase, and
vi) detecting and measuring a luminescence signal
is comprised by the invention.

Further, a method of determining the D-cysteine concentration in a cell or tissue sample comprising
i) lysing a tissue sample and adding a reducing agent to it;
ii) adding a functionalized or an unfunctionalized 6-hydroxy-2-cyanobenzothiazole or 6-amino-2-cyanobenzothiazole according to b) or d) of the precursors described above;
iii) adding luciferase;
iv) allowing the mixture obtained in step iii) to react;
v) detecting and measuring a luminescence signal;
vi) extrapolating from said signal the D-cysteine concentration;
is encompassed by the present invention.

The above method describes effectively a method of carrying out a new bioorthogonal click reaction forming a covalent bond of two molecules in biological and physiological environments, in vitro and in vivo.

It can be used in concert with the well-known Staudinger ligation. These two reactions are totally independent, which means that neither functionalized nor unfunctionalized 6-hydroxy-2-cyanobenzothiazole nor functionalized or unfunctionalized 6 - amino-2-cyanobenzothiazole nor functionalized or unfunctionalized D-cysteine can react with the Staudinger ligation reagents, i.e. modified phosphine and azides. This approach can allow the study or more complex systems or biological processes using the possibility of selectively modifying two different kinds of biomolecules at the same time, in vitro and in vivo.

Suitable reducing agents according to step i) are glutathione, thioredoxin, tris(2-carboxyethl)phosphine and dithiothreitol.The reducing agent serves to cleave disulfide bonds present in cell or tissue samples. Any D-cysteine that is potentially present will be set free.In steps ii. and iii. of the above method, functionalized or unfunctionalized 6-hydroxy-2-cyanobenzothiazole or 6-amino-2-cyanobenzothiazole and luciferase are added. 6-Hydroxy-2-cyanobenzothiazole or 6-amino-2-cyanobenzothiazole reacts with all forms of free cysteine such as L-, D- and N-terminal cysteine. A luminescent substrate is only obtained from the reaction with D-cysteine to give D-luciferin or amino-D-luciferin, which can then react with luciferase. Provided blocked 6-hydroxy-2-cyanobenzothiazole or blocked 6-amino-2-cyanobenzothiazole is used in step ii, biomolecules capable of unblocking these precursors will need to be present in the sample in order to obtain D-luciferin or amino-D-luciferin.

The concentration of D-cysteine can then be extrapolated from the luminescent signal.

This method of determining a D-cysteine concentration can specifically be used to determine the presence, concentration, location of bacteria, which express D-cysteine. In this way sepsis or any bacterial infection can be detected. In this way changes at the molecular level within deseased cells, i.e. the activities and concentrations of biomolecules present can be detected and help to develop selective therapeutics.

Another aspect of the invention involves a method for determining the presence of bacteria in food by
i) adding unfunctionalized or functionalized 6-hydroxy-2-cyanobenzothiazole or unfunctionalized or functionalized 6-amino-2-cyanobenzothiazole and unfunctionalized or functionalized D-cysteine to a sample to be investigated,
ii) optionally, adding luciferase,
iii) optionally, adding a magnesium cation source,
iv) allowing the components according to step i), optionally according to step i) and ii) or according to step i), ii) and iii), to react, and
v) detecting and measuring a luminescence signal.

This method also allows to test food samples for the presence of bacteria, as the reaction between D-luciferin or amino-D-luciferin and luciferase also requires adenosine triphosphate (ATP), which is normally absent in food unless living bacteria are present. The emitted light detected and measured is proportional to the amount of ATP present in the food. Thus the presence of living bacteria can be tested.

Yet another aspect of the invention is a method amenable to the study of isomerization of L- to D-luciferin, which is catalyzed by luciferase, in order to search for conditions and/or enzymes that can avoid this isomerization.

Also, protein or peptide degradation in cell or tissue samples or in organisms can be studied by using the method for determining D-cysteine concentration described above. In this case, step i. of the method would be omitted. Rather, any free D-cysteine present will have been released by degradation of the amino acid chains of proteins or peptides within the sample by processes such as autophagy. Addition of 6-hydroxy-2-cyanobezothiazole or 6-amino-2-cyanobenzothiazole in its unfunctionalised form will give rise to D-luciferin or amino-D-luciferin synthesis. Following the addition of luciferase, a luminescent signal can be detected and measures allowing the assessment of the degree of protein or peptide degradation occuring within the sample studied.

The bioorthogonal reactions described above could also be applied to fluorescent or radioactive studies of the glycosilation processes in the cell in vitro and in vivo. To this end, 6-hydroxy-2-cyanobenzothiazole or 6-amino-2-cyanobenzothiazole functionalized with a sugar moiety should be incubated with the cells or injected into animals. The cell glycan pathway incorporates the sugar derivative of 6-hydroxy-2-cyanobenzothiazole or 6-amino-2-cyanobenzothiazole as a normal sugar into different glycans. Following this, D-cysteine, an aminothiol or a 2-aminobenzylamine functionalized by fluorescent or radioactive labels are incubated with the cells or injected into the animals. Upon bioorthogonal reaction with the cyanobe nzothiazole moiety, labelling of glycans with fluorescent or radioactive label is achieved. Thus glycosilation processes in vitro and in vivo can be studied.

The invention is described in more detail hereinbelow, by means of illustrative figures and embodiments.
- **Fig. 1**: Condensation reaction between unfunctionalized 6-hydroxy-2-cyanobenzothiazole or 6-amino-2-cyanobenzothiazole and unfunctionalized D-cysteine yielding unfunctionalized D-luciferin or amino-D-luciferin.
- **Fig. 2**: Mechanism of the reaction yielding D-luciferin.
- **Fig. 3A**: Condensation reaction between a functionalized cyanobenzothiazole and a functionalized D-cysteine
- **Fig. 3B**: Condensation reaction between a functionalized cyanobenzothiazole and an aminothiol derivative

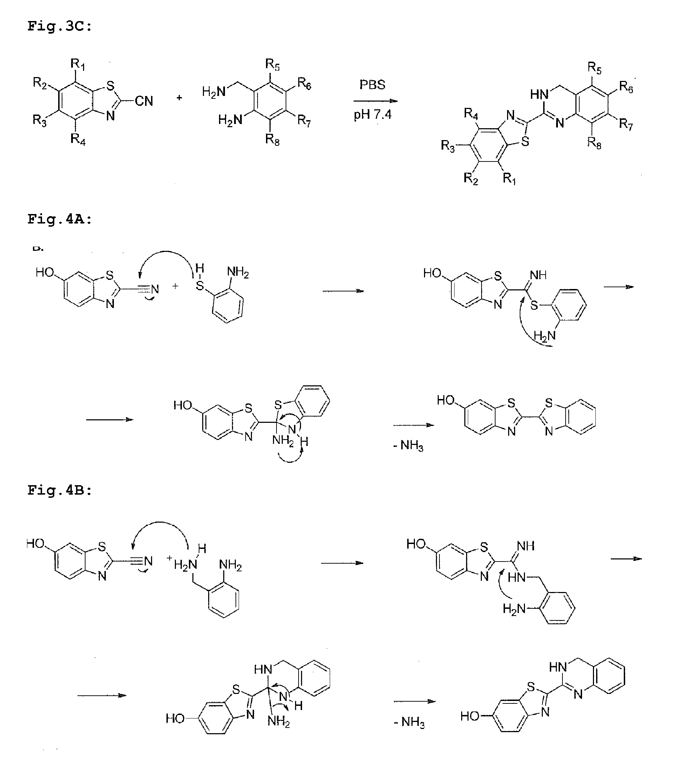

- **Fig. 3C**: Condensation reaction between a functionalized cyanobenzothiazole and a 2-aminobenzylamine
- **Fig 4A**: Reaction mechanism between 6-hydroxy-2-cyanobenzothiazole and an aminothiol derivative
- **Fig. 4B**: Reaction mechanism between 6-hydroxy-2-cyanobenzothiazole and 2-aminobenzylamine

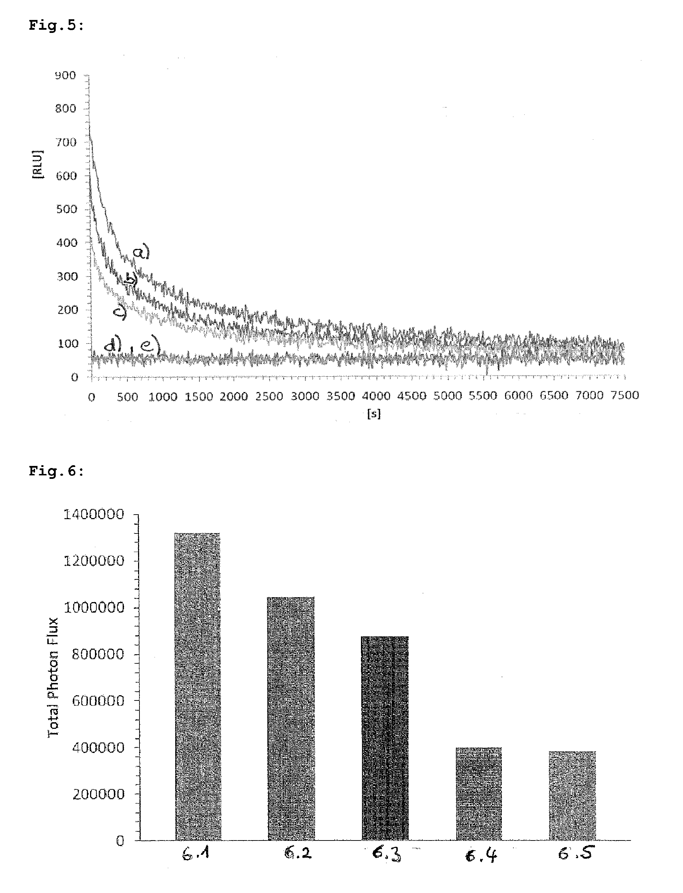

- **Fig. 5**: Graph showing the luminescence intensity [RLU] as a function of time [s] for solutions containing different concentrations of thrombin. Curves a) to e) depict results for the following mixtures, wherein CBT stands for cyanobenzothiazole: a) thrombin (20U) + peptide 1 + aminoCBT + luciferase b) thrombin (10U) + peptide 1 + aminoCBT + luciferase c) thrombin (5U) + peptide 1 + aminoCBT + luciferase d) peptide 1 + aminoCBT + luciferase e) luciferase assay buffer (control)
- **Fig. 6**: Diagram of total photon flux for solutions containing different concentrations of thrombin measured over a time period of 2h 4min 48s. Columns 6.1 to 6.5 represent the following samples, wherein CBT stands for cyanobenzothiazole: 6.1 thrombin (20U) + peptide1 + aminoCBT + luciferase 6.2 thrombin (10U) + peptide 1 + aminoCBT + luciferase 6.3 thrombin (5U) + peptide 1 + aminoCBT + luciferase 6.4 peptide 1 + aminoCBT + luciferase 6.5 luciferase assay buffer (control)

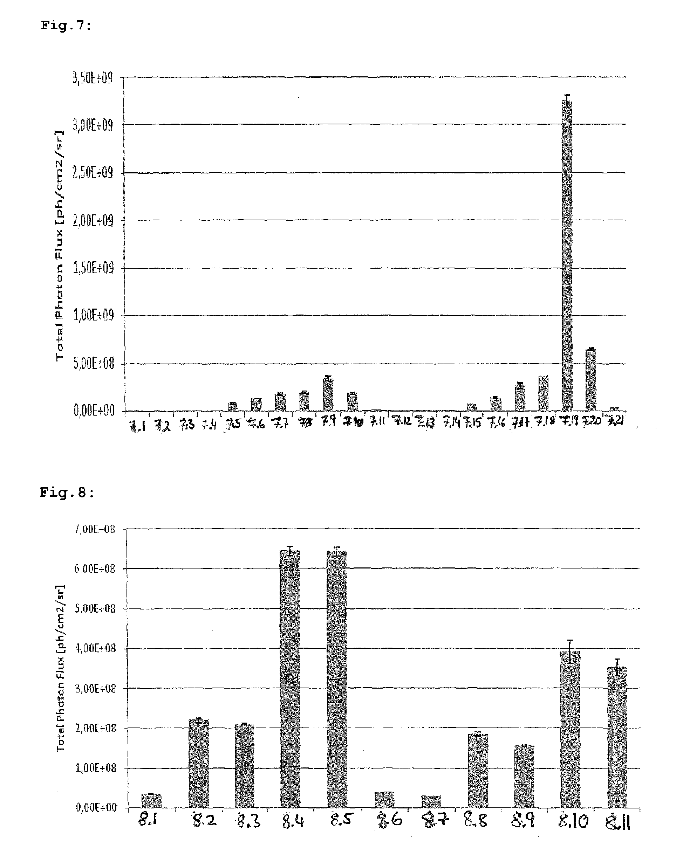

- **Fig. 7**: Diagram of total photon flux [ph/cm2/sr] for SKOV3-LUC-D3 living cell samples containing various combinations and concentrations of D-luciferin, OH-CBT, NH₂-CBT, D-Cys, L-Cys and NH₂-D-luciferin. Columns 7.1 to 7.21 correspond to the following sample solutions, wherein CBT stands for cyanobenzothiazole and Cys stands for cysteine: 7.1 NH₂-CBT (75 µM) 7.2 NH₂-CBT (75 µM), no washing step 7.3 NH₂-CBT (75 µM) + L-Cys (75 µM) 7.4 NH₂-CBT (0.75 µM) + D-Cys (0.75 µM) 7.5 NH₂-CBT (7.5 µM) + D-Cys (7.5 µM) 7.6 NH2-CBT (7.5 µM) + D-Cys (15 µM) 7.7 NH₂-CBT (7.5 µM) + D-Cys (37.5 µM) 7.8 NH2-CBT (7.5 µM) + D-Cys (75 µM) 7.9 NH₂-CBT (75 µM) + D-Cys (75 µM) 7.10 amino-luciferin (7.5 µM) 7.11 OH-CBT (75 µM) 7.12 OH-CBT (75 µM), no washing step 7.13 OH-CBT (75 µM) + L-Cys (75 µM) 7.14 OH-CBT (0.75 µM) + D-Cys (0.75 µM) 7.15 OH-CBT (7.5 µM) + D-Cys (7.5 µM) 7.16 OH-CBT (7.5 µM) + D-Cys (15 µM) 7.17 OH-CBT (7.5 µM) + D-Cys (37.5 µM) 7.18 OH-CBT (7.5 µM) + D-Cys (75 µM) 7.19 OH-CBT (75 µM) + D-Cys (75 µM) 7.20 luciferin (75 µM) 7.21 D-Cys (75 µM)
- **Fig. 8**: Diagram of total photon flux [ph/cm2/sr] for SKOV3-LUC-D3 living cell samples containing various combinations and concentrations of D-luciferin or amino-D-luciferin, D-Cys, L-Cys . Columns 8.1 to 8.11 correspond to the following sample solutions, wherein Cys stands for cysteine: 8.1 luciferin (0.75 µM) 8.2 luciferin (7.5 µM) 8.3 luciferin (7.5 µM) + D-Cys (7.5 µM) 8.4 luciferin (75 µM) 8.5 luciferin (75 µM) + D-Cys (75 µM) 8.6 aminoluciferin (0.75 µM) 8.7 aminoluciferin (0.75 µM) + D-Cys (0.75 µM) 8.8 aminoluciferin (7.5 µM) 8.9 aminoluciferin (7.5 µM) + D-Cys (7.5 µM) 8.10 aminoluciferin (75 µM) 8.11 aminoluciferin (75 µM) + D-Cys (75 µM)

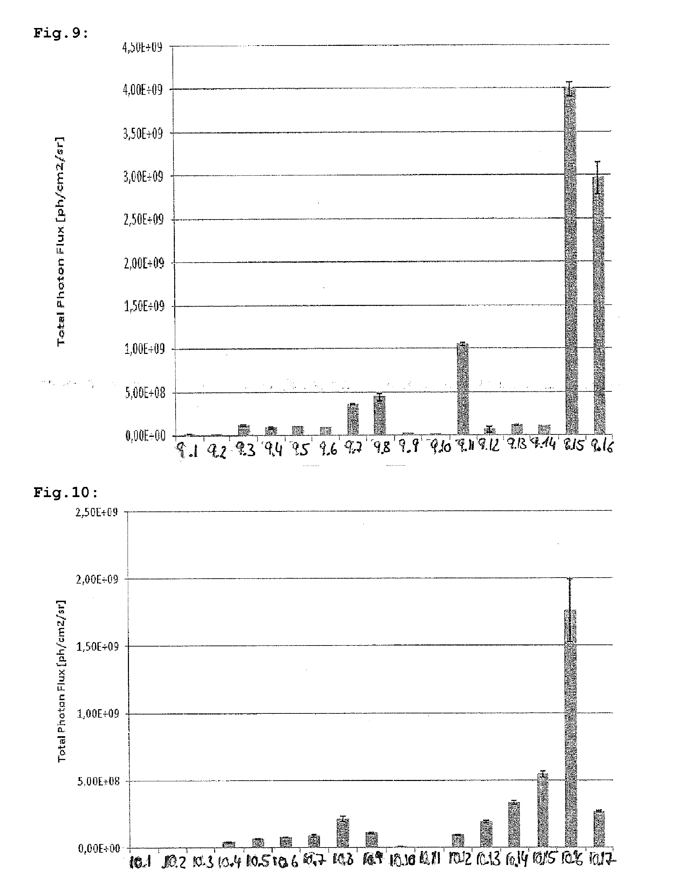

- **Fig. 9**: Diagram of total photon flux [ph/cm2/sr] for SKOV3-LUC-D3 living cell samples incubated with solutions of different concentrations of D-Cys, NH₂-CBT or OH-CBT for 20 min followed by a washing step with PBS and by addition of solutions of equal concentrations of complementary D-Cys, NH₂-CBT or OH-CBT, respectively. Columns 9.1 to 9.16 correspond to the following sample solutions, wherein CBT corresponds to cyanobenzothiazole and Cys to cysteine: 9.1 D-Cys (7.5 µM) 20min incubated, washed + NH₂-CBT (7.5 µM) 9.2 NH₂-CBT (7.5 µM) 20min incubated, washed + D-Cys (7.5 µM) 9.3 D-Cys (75 µM) 20min incubated, washed + NH₂-CBT (75 µM) 9.4 NH₂-CBT (75 µM) 20min incubated, washed + D-Cys (75 µM) 9.5 D-Cys (7.5 µM) 20min incubated, no washing step + NH₂-CBT (7.5 µM) 9.6 NH₂-CBT (7.5 µM) 20min incubated, no washing step + D-Cys (7.5 µM) 9.7 D-Cys (75 µM) 20min incubated, no washing step + NH₂-CBT (75 µM) 9.8 NH₂-CBT (75 µM) 20min incubated, no washing step + D-Cys (75 µM) 9.9 D-Cys (7.5 µM) 20min incubated, washed + OH-CBT (7.5 µM) 9.10 OH-CBT (7.5 µM) 20min incubated, washed + D-Cys (7.5 µM) 9.11 D-Cys (75 µM) 20min incubated, washed + OH-CBT (75 µM) 9.12 OH-CBT (75 µM) 20min incubated, washed + D-Cys (75 µM) 9.13 20min incubated, no washing step + OH-CBT (7.5 µM) 9.14 OH-CBT (7.5 µM) 20min incubated, no washing step + D-Cys (7.5 µM) 9.15 D-Cys (75 µM) 20min incubated, no washing step + OH-CBT (75 µM) 9.16 OH-CBT (75 µM) 20min incubated, no washing step + D-Cys (75 µM) **Fig. 10** Diagram of total photon flux [ph/cm2/sr] for MDA-MB-231-Luc living cell samples containing various combinations and concentrations of D-luciferin, amino-D-luciferin, OH-CBT, NH₂-CBT, D-Cys and L-Cys. Columns 10.1 to 10.17 correspond to the following sample solutions, wherein CBT stands for cyanobenzylthiazole and Cys stands for cysteine.: 10.1 D-Cys (75 µM) 10.2 NH₂-CBT (75 µM) 10.3 NH₂-CBT (75 µM) + L-Cys (75 µM) 10.4 NH2-CBT (7.5 µM) + D-Cys (7.5 µM) 10.5 NH₂-CBT (7.5 µM) + D-Cys (15 µM) 10.6 NH2-CBT (7.5 µM) + D-Cys (37.5 µM) 10.7 NH2-CBT (7.5 µM) + D-Cys (75 µM) 10.8 NH₂-CBT (75 µM) + D-Cys (75 µM) 10.9 aminoluciferin (75 µM) 10.10 OH-CBT (75 µM) 10.11 OH-CBT (75 µM) + L-Cys (75 µM) 10.12 OH-CBT (7.5 µM) + D-Cys (7.5 µM) 10.13 OH-CBT (7.5 µM) + D-Cys (15 µM) 10.14 OH-CBT (7.5 µM) + D-Cys (37.5 µM) 10.15 OH-CBT (7.5 µM) + D-Cys (75 µM) 10.16 OH-CBT (75 µM) + D-Cys (75 µM) 10.17 luciferin (75 µM)

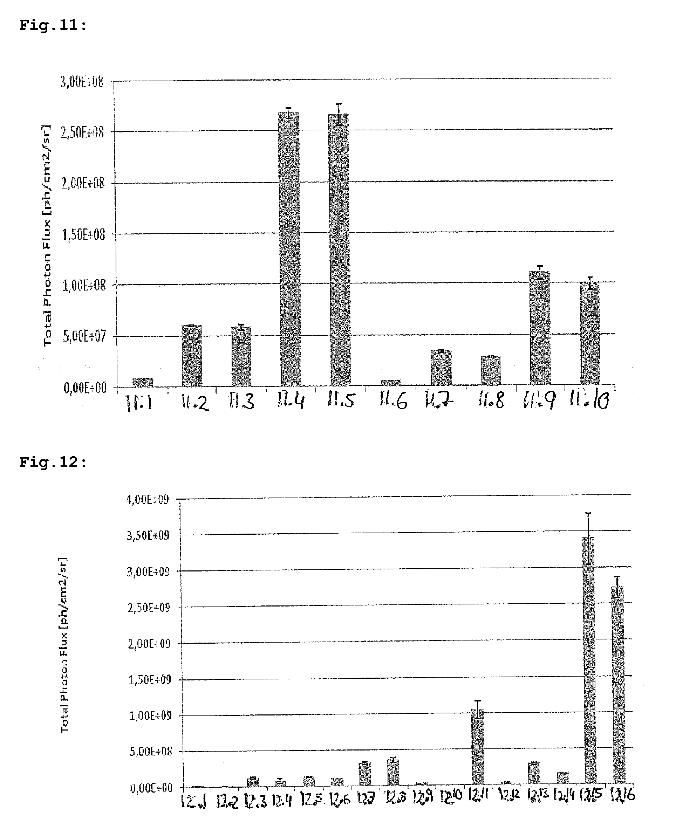

- **Fig. 11**: Diagram of total photon flux [ph/cm2/sr] for MDA-MB-231-Luc living cell samples containing various combinations and concentrations of D-luciferin, amino-D-luciferin and D-Cys. Columns 11.1 to 11.10 correspond to the following sample solutions, wherein Cys stands for cysteine: 11.1 luciferin (0.75 µM) 11.2 luciferin (7.5 µM) 11.3 luciferin (7.5 µM) + D-Cys (7.5 µM) 11.4 luciferin (75 µM) 11.5 luciferin (75 µM) + D-Cys (75 µM) 11.6 amino-D-luciferin (0.75 µM) 11.7 amino-D-luciferin (7.5 µM) 11.8 amino-D-luciferin (7.5 µM) + D-Cys (7.5 µM) 11.9 amino-D-luciferin (75 µM) 11.10 amino-D-luciferin (75 µM) + D-Cys (75 µM)
- **Fig. 12**: Diagram of total photon flux [ph/cm2/sr] for MDA-MB-231-Luc living cell samples incubated with solutions of different concentrations of D-Cys, NH₂-CBT or OH-CBT for 20 min followed by a washing step with PBS and by addition of solutions of equal concentrations of complementary D-Cys, NH₂-CBT or OH-CBT, respectively. Columns 12.1 to 12.16 correspond to the following sample solutions, wherein Cy stands for cysteine and CBT stands for cyanobenzothiazole: 12.1 D-Cys (7.5 µM) + 20 min incubated, washed + NH₂-CBT (7.5 µM) 12.2 NH₂-CBT (7.5 µM) + 20 min incubated, washed + D-Cys (7.5 µM) 12.3 D-Cys (75 µM) + 20 min incubated, washed + NH₂-CBT (75 µM) 12.4 NH₂-CBT (75 µM) + 20 min incubated, washed + D-Cys (75 µM) 12.5 D-Cys (7.5 µM) + 20 min incubated, no washing step + NH₂-CBT (7.5 µM) 12.6 NH₂-CBT (7.5 µM) 20 min incubated, no washing step + D-Cys (7.5 µM) 12.7 D-Cys (75 µM) 20 min incubated, no washing step + NH₂-CBT (75 µM) 12.8 NH₂-CBT (75 µM) 20 min incubated, no washing step + D-Cys (75 µM) 12.9 D-Cys (7.5 µM) + 20 min incubated, washed + OH-CBT (7.5 µM) 12.10 OH-CBT (7.5 µM) 20 min incubated, washed + D-Cys (7.5 µM) 12.11 D-Cys (75 µM) 20 min incubated, washed + OH-CBT (75 µM) 12.12 OH-CBT (75 µM) 20 min incubated, washed + D-Cys (75 µM) 12.13 D-Cys (7.5 µM) 20 min incubated, no washing step + OH-CBT (7.5 µM) 12.14 OH-CBT (7.5 µM) 20 min incubated, no washing step + D-Cys (7.5 µM) 12.15 D-Cys (75 µM) 20 min incubated, no washing step + OH-CBT (75 µM) 12.16 OH-CBT (75 µM) 20 min incubated, no washing step + D-Cys (75 µM)

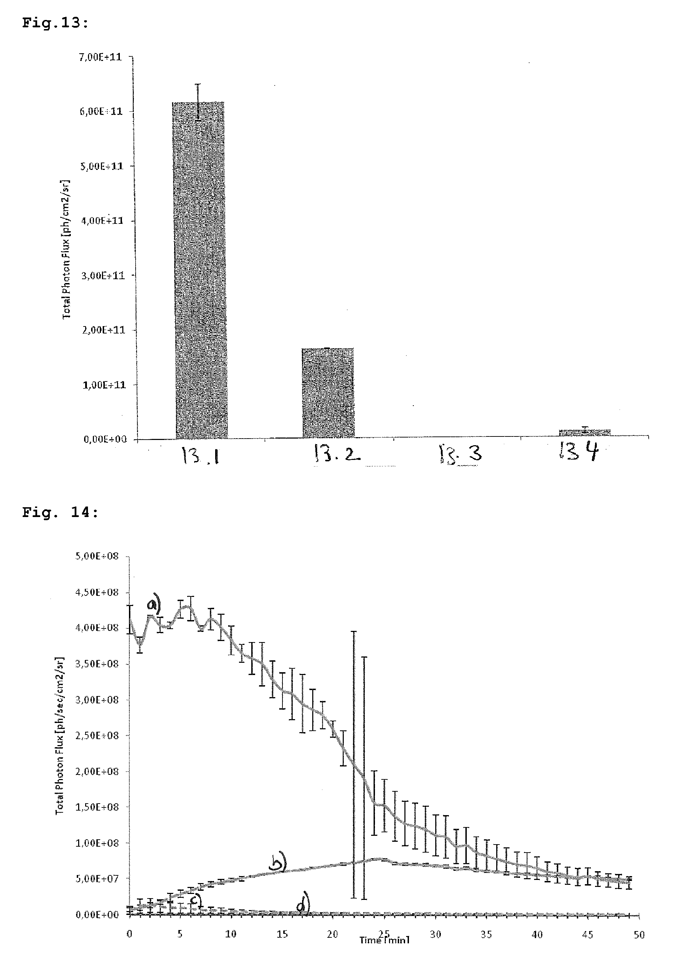

- **Fig. 13**: Diagram of total photon flux [ph/cm2/sr] for FVB-luc+ healthy mice with ubiquitous D-luciferase expression injected intraperitoneally with either D-luciferin or D-Cys + OH-CBT or OH-CBT only or with a control solution. Columns 13.1 to 13.4 correspond to the following sample solutions, wherein CBT stands for caynobezothiazole, Cys for cysteine and PBS and DMS for phosphate buffered saline and dimethylsulfide, respectively: 13.1 luciferin 13.2 OH-CBT + D-Cys 13.3 PBS + DMS (control solution) 13.4 OH-CBT
- **Fig. 14**: Graph of time vs. total photon flux [ph/cm2/sr] for FVB-luc+ healthy mice with ubiquitous D-luciferase expression injected intraperitoneally with either D-luciferin or D-Cys + OH-CBT or OH-CBT only or with a control solution. Curves a) to d) represent, wherein CBT stands for cyanobezothiazole and Cys for cysteine and PBS and DMS for phosphate buffered saline and dimethylsulfide, respectively: a) luciferin b) b) OH-CBT + D-Cys c) OH-CBT d) PBS + DMS (control)

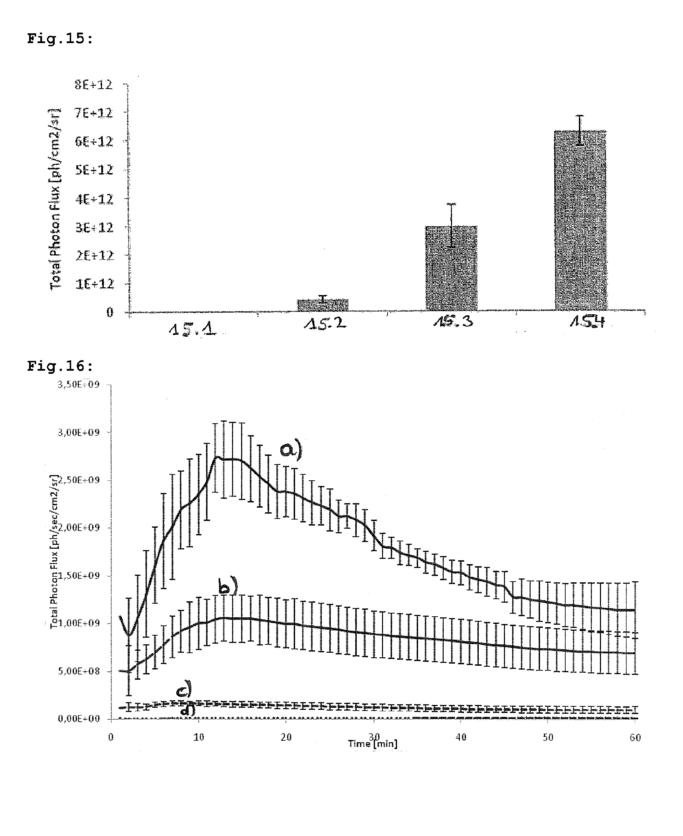

- **Fig. 15**: Diagram of total photon flux [ph/cm2/sr] for FVB-luc+ healthy mice with ubiquitous D-luciferase expression injected intraperitoneally first with either a phosphate buffered saline (PBS) solvent sample or D-Cys, followed by another injection with either D-luciferin or OH-CBT. Columns 15.1 to 15.4 correspond to the following sample solutions, wherein CBT stands for cyanobezothiazole and Cys for cysteine, respectively: 15.1 OH-CBT + PBS 15.2 OH-CBT + D-Cys (1:1) 15.3 OH-CBT + D-Cys (1:10) 15.4 D-luciferin
- **Fig. 16**: Graph of time vs. total photon flux [ph/cm2/sr] for FVB-luc+ healthy mice with ubiquitous D-luciferase expression injected intraperitoneally first with either a phosphate buffered saline (PBS) solvent sample or D-Cys, followed by another injection with either D-luciferin or OH-CBT. Curves a) to d) represent the following, wherein CBT stands for cyanobezothiazole and Cys for cysteine: a) D-luciferin b) OH-CBT + D-Cys (1:10) c) OH-CBT + D-Cys (1:1) d) OH-CBT

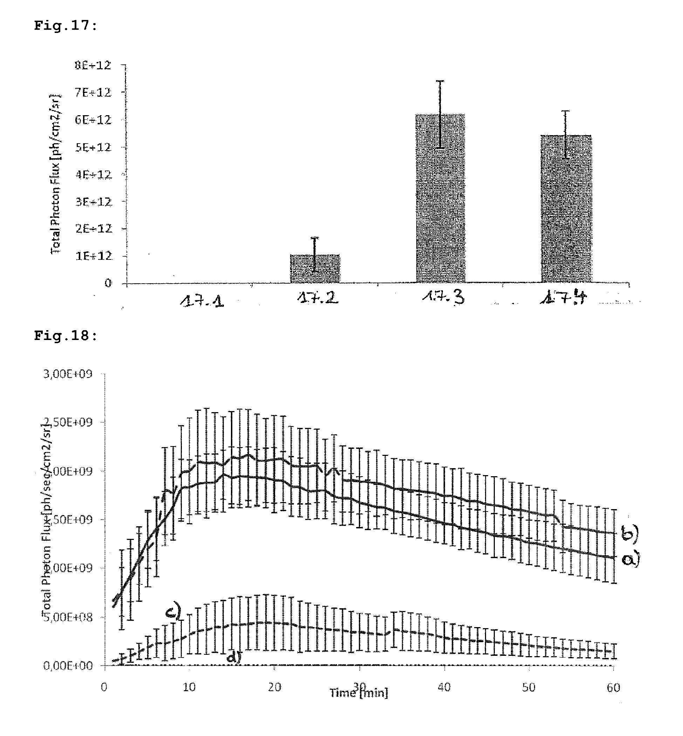

- **Fig. 17**: Diagram of total photon flux [ph/cm2/sr] for FVB-luc+ healthy mice with ubiquitous D-luciferase expression injected intraperitoneally first with either a phosphate buffered saline (PBS) solvent sample or D-Cys, followed by another injection with either 6-amino-2-cyanobenzothiazole (NH₂-CBT), amino-D-luciferin or a solvent control sample. Columns 17.1 to 17.4 correspond to the following sample solutions, wherein CBT stands for cyanobezothiazole and Cys for cysteine, respectively: 17.1 NH2-CBT 17.2 NH₂-CBT + D-Cys (1:1) 17.3 NH₂-CBT+ D-Cys (1:10) 17.4 Amino-D-luciferin
- **Fig. 18**: Graph of time vs. total photon flux [ph/cm2/sr] for FVB-luc+ healthy mice with ubiquitous D-luciferase expression injected intraperitoneally first with either a phosphate buffered saline (PBS) solvent sample or D-Cys, followed by another injection with either 6-amino-2-cyanobenzothiazole (NH₂-CBT), amino-D-luciferin or a solvent control sample. Curves a) to d) represent, wherein CBT stands for cyanobezothiazole and Cys for cysteine: a) Amino-D-luciferin b) NH₂-CBT + D-Cys (1:10) c) NH₂-CBT + D-Cys (1:1) d) NH₂-CBT

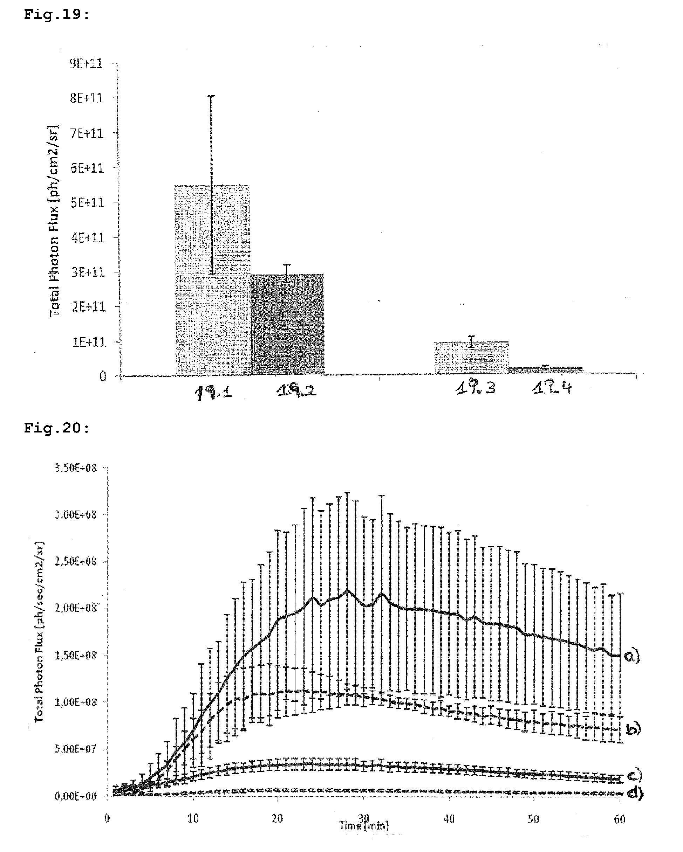

- **Fig. 19**: Diagram of total photon flux [ph/cm2/sr] for FVB-luc+ healthy mice with ubiquitous D-luciferase expression, where the columns represent: 19.1 the total photon flux of a group of four mice injected with lipopolysaccharide (LPS) and D-(+)-Galactosamine (D-GaIN), followed after 6 hours by an injection with DEVD-amino-D-luciferin 19.2 the total photon flux of a group of four mice injected with the solvent vehicle (PBS), followed after 6 hours by an injection with DEVD-amino-D-luciferin 19.3 the total photon flux of a group of four mice injected with lipopolysaccharide (LPS) and D-(+)-Galactosamine (D-GaIN), followed after 6 hours by An injection with DEVD-D-Cys, then NH₂-CBT 19.4 the total photon flux of a group of four mice injected with the solvent vehicle (PBS), followed after 6 hours by an injection with DEVD-D-Cys, then NH₂-CBT
- **Fig. 20**: Graph of time vs. total photon flux [ph/cm2/sr] for FVB-luc+ healthy mice with ubiquitous D-luciferase expression injected intraperitoneally with a) lipopolysaccharide (LPS) and D-(+)-Galactosamine (D-GaIN), followed after 6 hours by an injection with DEVD-amino-D-luciferin b) lipopolysaccharide (LPS) and D-(+)-Galactosamine (D-GaIN), followed after 6 hours by an injection with DEVD-D-Cys, then NH₂-CBT c) the solvent vehicle (PBS), followed after 6 hours by an injection with DEVD-amino-D-luciferin d) the solvent vehicle (PBS), followed after 6 hours by an injection with DEVD-D-Cys, then NH₂-CBT

### Example 1 - Determination of the concentration of an enzyme by using functionalized D-cysteine as a precursor to D-luciferin

A 1000 U/ml stock solution of thrombin (product number T1063, from human plasma, Sigma-Aldrich) in storage buffer (50mM sodium citrate pH 6.5, 200mM NaCl, 0.1% PEG-6000, 50% Glycerol) was prepared.

The solution containing Gly-Gly-Arg-DCys (410 µL, peptide 1) and thrombin (200 U/mL) in cleavage buffer (20mM Tris-HCL pH 8.4, 150mM NaCl, 2.5mM CaCl₂) was incubated for three hours at room temperature. The same was done for a 100 U/mL and a 50 U/mL solution of thrombin. 100 µL portions of each of the three solutions were mixed with 2-cyano-6-aminobenzothiazole (amino CBT, 17.08 µL) solution in 2:1 EtOH/MeOH solvent. The mixtures were kept under an air atmosphere at room temperature for 4 hours.

5 µL portions of each of the solutions were then diluted with luciferase assay buffer (0.1mM Tris-HCl pH 8.0, 2mM adenosine triphosphate (ATP), 5mM MgSO₄) and mixed with a 60 µg/mL D-luciferase solution (100 µL, D-luciferase from Photinuspyralis (firefly) product No. L9506, Sigma-Aldrich) in luciferase assay buffer.

Luminescent intensity in relative light units (RLU) was measured immediately with a luminometer for an integration period of 13 seconds.

The luminescent intensity as well as the total photon flux were the highest for the solution containing the largest thrombin concentration and became smaller when this concentration decreased. The luminescent intensity and the total photon flux was then proportional to the thrombin concentration. When no thrombin is present, the luminescent signal is the same as for the blank (luciferase assay buffer without any luciferase substrate). The luminescent signal obtained for solutions containing thrombin were significantly higher than for the ones that do not contain thrombin.

The results of this experiment are also depicted in Fig.5 and 6.

### Example 2a - Determination of the presence of D-luciferase in SKOV3-Luc-D3 living cells

Luciferase-expressing SKOV3-Luc-D3 cells were grown in 10 cm round culture plates with McCoy's media (Gibco) containing 10% fetal bovine serum (FBS), penicillin/streptomycin and glutamax. On a black 96-well-plate, 10000 cells per well were seeded. After 48 hours the cells were first washed with phosphate buffered saline (PBS, 200 µL) and then incubated for 5 minutes in a solution containing D-cysteine (D-Cys) or PBS (100 µL).

Then a solution of 6-Hydroxy-2-cyanobenzothiazole (OH-CBT), 6-amino-2-cyanobenzothaizole (NH₂-CBT) or D-luciferin (100 µL) was added immediately before imaging using an IVIS spectrometer.

For each reagent, solutions of three different concentrations were used, namely 150 µM, 15 µM and 1.5 µM, giving rise to final concentrations for all reagents of 75 µM, 7.5 µM and 0.75 µM.

The following reagent combinations were used:
● NH₂-CBT (75µM) + D-Cys(75µM)
● NH₂-CBT (7.5µM) + D-Cys (7.5µM)
● NH₂-CBT (0.75µM) + D-Cys (0.75µM)
● NH₂-CBT (7.5µM) + D-Cys (15µM)
● NH₂-CBT (7.5µM) + D-Cys (37.5µM)
● NH₂-CBT (7.5µM) + D-Cys (75µM)
● NH₂-CBT (75µM) + L-Cys(75µM)
● NH₂-CBT (75µM)
● D-Luciferin (75µM)
● D-Luciferin (7.5µM)
● D-Luciferin (0.75µM)
● D-Luciferin (7.5µM) + D-Cys (7.5µM)
● D-Luciferin (75µM) + D-Cys (75µM)
● D-Cys (75µM)
● OH-CBT (75µM) + D-Cys(75µM)
● OH-CBT (7.5µM) + D-Cys (7.5µM)
● OH-CBT (0.75µM) + D-Cys (0.75µM)
● OH-CBT (7.5µM) + D-Cys (15µM)
● OH-CBT (7.5µM) + D-Cys (37.5µM)
● OH-CBT (7.5µM) + D-Cys (75µM)
● OH-CBT (75µM) + L-Cys(75µM)
● OH-CBT (75µM)
● amino-D-Luciferin (75µM)
● amino-D-Luciferin (7.5µM)
● amino-D-Luciferin (0.75µM)
● amino-D-Luciferin (7.5µM) + D-Cys (7.5µM)
● amino-D-Luciferin (75µM) + D-Cys (75µM)
● PBS

Each of the experiments with the above reagent combinations was performed in triplicate.

Immediately after addition of the OH-CBT, NH₂-CBT or D-luciferin solutions, IVIS spectra were recorded every minute for one hour on an IVIS spectrometer using living image software on auto settings.

The results recorded are shown in Fig.7 and 8.

### Example 2b

In order to determine if the reactions of Example 2a above occurred inside or outside the cells, the cells were first incubated with either D-Cys or OH-CBT or NH₂-CBT (75 or 7.5 µL) for 20 minutes. The cells were then washed with PBS. Following this, OH- or NH₂-CBT or D-Cys solutions of 75 µL or 7.5 µL concentrations were added.

The following reagent combinations were used with "20 min" indicating the reagent with which the cells were incubated over the 20 minute period:
● NH₂-CBT 20min (7.5µM) + D-Cys(7.5µM)
● NH₂-CBT 20min (75µM) + D-Cys (75µM)
● D-Cys 20min (7.5µM) + NH₂-CBT (7.5µM)
● D-Cys 20min(75µM) + NH₂-CBT(75µM)
● OH-CBT 20min (7.5µM) + D-Cys (7.5µM)
● OH-CBT 20min (75µM) + D-Cys(75µM)
● D-Cys 20min (7.5µM) + OH-CBT (7.5µM)
● D-Cys 20min (75µM) + OH-CBT (75µM)

Each of the experiments with the above reagent combinations was performed in triplicate.

Immediately after addition of the OH-CBT, NH₂-CBT or D-Cys solutions to the incubated cells, IVIS spectra were recorded every minute for one hour on an IVIS spectrometer using living image software on auto settings.

The results recorded are shown in Fig.9.

### Example 2c

The experiments described in Example 2b were repeated without the washing step.

The results recorded are shown in Fig.9.

### Example 3a - Determination of the presence of luciferase in MDA-MB-231-Luc living cells

D-Luciferase-expressing MDA-MB-231-Luc cells were grown in 10cm round culture plates with MEM alpha media (Gibco) containing 10% fetal bovine serum, penicilline/strepomycin and glutamax.

On a black 96-well plate, 10000 cell/well were seeded. 48h after the seeding, the cells were first washed with phosphate buffer solution (200 µL) and then incubated for 5 min in solutions containing D-Cys or PBS (100 µL).

Then a solution of OH-CBT, NH₂-CBT or D-luciferin (100 µL) was added.

Solutions of three different concentrations for each reagent were used, namely 150 µM, 15 µM and 1.5 µM, giving rise to final concentrations of 75 µM, 7.5 µM or 0.75 µM for all the reagents.

The following reagent combinations were used:
● NH₂-CBT (75µM) + D-Cys(75µM)
● NH₂-CBT (7.5µM) + D-Cys (7.5µM)
● NH₂-CBT (0.75µM) + D-Cys (0.75µM)
● NH₂-CBT (7.5µM) + D-Cys (15µM)
● NH₂-CBT (7.5µM) + D-Cys (37.5µM)
● NH₂-CBT (7.5µM) + D-Cys (75µM)
● NH₂-CBT (75µM) + L-Cys(75µM)
● NH₂-CBT (75µM)
● D-Luciferin (75µM)
● D-Luciferin (7.5µM)
● D-Luciferin (0.75µM)
● D-Luciferin (7.5µM) + D-Cys (7.5µM)
● D-Luciferin (75µM) + D-Cys (75µM)
● D-Cys (75µM)
● OH-CBT (75µM) + D-Cys(75µM)
● OH-CBT (7.5µM) + D-Cys (7.5µM)
● OH-CBT (0.75µM) + D-Cys (0.75µM)
● OH-CBT (7.5µM) + D-Cys (15µM)
● OH-CBT (7.5µM) + D-Cys (37.5µM)
● OH-CBT (7.5µM) + D-Cys (75µM)
● OH-CBT (75µM) + L-Cys(75µM)
● OH-CBT (75µM)
● amino-D-Luciferin (75µM)
● amino-D-Luciferin (7.5µM)
● amino-D-Luciferin (0.75µM)
● amino-D-Luciferin (7.5µM) + D-Cys (7.5µM)
● amino-D-Luciferin (75µM) + D-Cys (75µM)
● PBS

Each of the experiments with the above reagent combinations was performed in triplicate.

Immediately after addition of the D-Cys or L-Cys solutions, IVIS spectra were recorded every minute for one hour on an IVIS spectrometer using living image software on auto settings.

The results recorded are shown in Fig.10 and 11.

### Example 3b

In order to determine if the reactions of Example 3a above occurred inside or outside the cells, the cells were first incubated with either D-Cys or OH-CBT or NH₂-CBT (75 or 7.5 µL) for 20 minutes. The cells were then washed with PBS. Following this, OH- or NH₂-CBT or D-Cys solutions of 75 µL or 7.5 µL concentrations were added.

The following reagent combinations were used with "20 min" indicating the reagent with which the cells were incubated over the 20 minute period:
● NH₂-CBT 20min (7.5µM) + D-Cys(7.5µM)
● NH₂-CBT 20min (75µM) + D-Cys (75µM)
● D-Cys 20min (7.5µM) + NH₂-CBT (7.5µM)
● D-Cys 20min (75µM) + NH₂-CBT (75µM)
● OH-CBT 20min (7.5µM) + D-Cys (7.5µM)
● OH-CBT 20min (75µM) + D-Cys (75µM)
● D-Cys 20min (7.5µM) + OH-CBT (7.5µM)
● D-Cys 20min (75µM) + OH-CBT (75µM)

Each of the experiments with the above reagent combinations was performed in triplicate.

Immediately after addition of the OH-CBT, NH₂-CBT or D-Cys solutions to the incubated cells, IVIS spectra were recorded every minute for one hour on an IVIS spectrometer using living image software on auto settings.

The results recorded are shown in Fig.12.

### Example 3c

The experiments described in Example 3b were repeated without the washing step.

The results recorded are shown in Fig.12.

### Example 4a - Determination of the presence of luciferase in FVB-luc+ healthy mice with ubiquitous luciferase expression

Four groups of two healthy FVB-luc+ mice ((FVB-Tg(CAG-luc-GFP)L2G85Chco/J) mice) each expressing ubiquitously luciferase were injected intraperitoneally with either a 30 mg/mL solution of D-luciferin (150 mg/kg or 0.535 mmol/kg) or with a 12.966 mg/mL solution of D-Cys (64.83 mg/kg) in PBS followed by a 60 mg/mL solution of OH-CBT (94.28 mg/kg or 0.535 mmol/kg) in DMSO three minutes later or with a 60 mg/mL solution of OH-CBT (94.28 mg/kg or 0.535 mmol/kg) in DMSO only or a solvent control sample of PBS and DMSO only, which contained the same solvent volume as the other samples depending on mouse weight.

The animals were anesthetized using isofluorane and one image per minute was recorded over a period of 50 minutes for each mouse using a Caliper life science IVIS spectrometer on auto settings. The data were treated with living image software (Caliper Life Science) using the ROI tool.

The results recorded are depicted in Fig.13 and 14.

### Example 4b - Determination of the presence of luciferase in healthy FVB-luc+ mice with ubiquitous luciferase expression

A group of five healthy FVB-Luc+ mice ((FVB-Tg(CAG-luc-GFP)L2G85Chco/J) mice), each expressing ubiquitously luciferase, were injected intraperitoneally with D-cysteine (D-Cys) (0.268 mmol/kg), which was formulated as a 0.0536 mmol/mL solution in phosphate buffered saline (PBS). A second group of the same type and number of mice was injected with a ten-fold amount of D-Cys, namely 2.68 mmol/kg. Two further groups of five mice each were injected with a PBS control sample.

Three minutes later, the same mice were injected with either 6-hydroxy-2-cyanobenzothiazole (OH-CBT), or D-luciferin or a PBS or dimethylsulfoxide (DMSO) solvent control sample. Each of these samples was administered in an amount of 0.268 mmol/kg. D-luciferin was formulated in PBS at a concentration of 0.0536 mmol/mL and OH-CBT was formulated in DMSO at a concentration of 0.268 mmol/mL.

Immediately after the second injection, luminescence images were acquired using a Xenon IVIS Spectrum imaging system (Caliper Life Science, Hopkinton, MA, USA). One image per minute over a period of 60 minutes was recorded using the automatic setting available on the instrument.

The results recorded are shown in Fig. 15 and Fig.16.

### Example 4c

The same experiment as described in example 4b was repeated using in the second injection step 0.268 mmol/kg of either 6-amino-2-cyanobenzothiazole (NH₂-CBT), amino-D-luciferin or a PBS or dimethylsulfoxide (DMSO) solvent control sample. NH₂-CBT was formulated in DMSO at a concentration of 0.268 mmol/mL.

Immediately after the second injection, luminescence images were acquired using a Xenon IVIS Spectrum imaging system (Caliper Life Science, Hopkinton, MA, USA). One image per minute over a period of 60 minutes was recorded using the automatic setting available on the instrument.

The results are shown in Fig. 17 and Fig. 18.

### Example 5

Two groups of four healthy FVB-Luc+ mice ((FVB-Tg(CAG-luc-GFP)L2G85Chco/J) mice), each expressing ubiquitously luciferase, were injected with 100 µg/kg of a lipopolysaccharide (LPS) solution in phosphate buffered saline (PBS) of a concentration of 3 µg LPS in 50 µL PBS and with 267 mg/kg of a solution of D-(+)-Galactosamine (D-GaIN) in PBS of a concentration of 8 mg in 50 µL PBS in order to induce hepatic apoptosis and thus Capase-3/7 activation.

After six hours, 22 mg/kg of a DEVD-D-Cysteine (DEVD-D-Cys) in PBS solution having a concentration of 0.68 mg/100 µL was injected intraperitoneally into the mice belonging to one of the groups of mice treated with LPS and D-GaIN, followed 10 minutes later by 6.8mg/kg of a 6-amino-2-cyanobenzothiazole (NH₂-CBT) in DMSO solution having a concentration of 0.205 mg/20 µL.

As a positive control, 34 mg/kg of a DEVD-amino-D-luciferin solution in PBS having a concentration of 1.02 mg/100 µL was injected into the other group of mice treated with LPS and D-GaIN after six hours instead of DEVD-D-Cysteine (DEVD-D-Cys) and 6-amino-2-cyanobenzothiazole (NH₂-CBT).

Another two groups of four untreated FVB-Luc+ mice each were injected with 22 mg/kg of a DEVD-D-Cysteine (DEVD-D-Cys) in PBS solution having a concentration of 0.68 mg/100 µL, followed by 6.8mg/kg of a 6-amino-2-cyanobenzothiazole (NH₂-CBT) in DMSO solution having a concentration of 0.205 mg/20 µL and with 34 mg/kg of a DEVD-amino-D-luciferin solution in PBS having a concentration of 1.02 mg/100 µL, respectively.

The presence of capase-3/7 activity in the mice is indicated by cleavage of the DEVD-peptide off DEVD-D-Cys or DEVD-amino-D-luciferin followed by luminescence arising from the reaction between luciferase and amino-D-luciferin.

A Xenogen IVIS Spectrum imaging system (Caliper Life Science, Hopkinton, MA, USA) was used to acquire luminescence images. One image per minute over a period of 60 minutes was recorded using the automatic setting available on the instrument.

The results are shown in Fig. 19 and Fig. 20.

Also, apart from studying the protease activity of capase-3/7 in cell death signalling, the activities of other proteases in cell death signalling or upon metabolic change can be studied in vivo and in vitro. To this end, a reaction of either 6-hydroxy-2-cyanobenzothiazole or 6-amino-2-cyanobenzothiazole with the D-cysteine released from the C-terminal of a peptide sequence specific to the protease of interest is studied and controlled via the bioluminescent signal. Only if D-cysteine is released by the specific protease, it can react with 6-hydroxy-2-cyanobenzthiazole or 6-amino-2-cyanobenzothiazole, giving rise to D-luciferin or amino-D-luciferin, i.e. the substrate for luciferase.

The following table shows the proteases whose activities can be studied and controlled and the respective imaging kits comprising either 6-hydroxy-2-cyanobenzothiazole (6-OH-CBT) or 6-amino-2-cyanobenzothiazole(6-NH2-CBT)and a D-cysteine attached to a peptide sequence, which is a cleavage site for the specific protease studied:

| **Imaging kit** | **Protease** |
|---|---|
| Z-DEVD-dCys + 6-OH-CBT or 6-NH2-CBT | caspase-3 and -7 |
| Z-LETD-dCys + 6-OH-CBT or 6-NH2-CBT | caspase-8 |
| Z-LEHD-dCys + 6-OH-CBT or 6-NH2-CBT | caspase-9 |
| GP-dCys + 6-OH-CBT or 6-NH2-CBT | dipeptidyl peptidase 4 (DPPIV) |
| Suc-dCys + 6-OH-CBT or 6-NH2-CBT | calpain- and chymotrypsin-like activities of proteasome |
| Z-LRR-dCys + 6-OH-CBT or 6-NH2-CBT | trypsine-like activity of proteasome |
| Z-nLPnLD-dCys + 6-OH-CBT or 6-NH2-CBT | caspase-like activity of proteasome |
| Z-QEVY-dCys + 6-OH-CBT or 6-NH2-CBT | clapain and proteasome chymotrypsine-like activity |
| VP-dCys + 6-OH-CBT or 6-NH2-CBT | dipeptidyl peptidase 4 (DPPIV) |
| Z-VDAVD-dCys + 6-OH-CBT or 6-NH2-CBT | caspase-2 |
| Z-VEID-dCys + 6-OH-CBT or 6-NH2-CBT | caspase-6 |
| Z-ATAD-dCys + 6-OH-CBT or 6-NH2-CBT | caspase-12 |
| Z-IEPD-dCys + 6-OH-CBT or 6-NH2-CBT | granzyme B |
| Z-IETD-dCys + 6-OH-CBT or 6-NH2-CBT | granzyme B and caspase-6 |
| Z-TSAVLQ-dCys + 6-OH-CBT or 6-NH2-CBT | SARS protease |
| Z-VNSTLQ-dCys + 6-OH-CBT or 6-NH2-CBT | SARS protease |

## Claims

1. A precursor molecule for the synthesis of D-luciferin, amino-D-luciferin, blocked D-luciferin or blocked amino-D-luciferin, chosen from the group consisting of
a) functionalized D-cysteine, wherein the functionalization does not prevent the reactivity towards 6-hydroxy-2-cyanobenzothiazole or 6-amino-2-cyanobenzothiazole; and wherein, when reacting with a molecule according to b) or when reacting with an unfunctionalized 6-hydroxy-2-cyanobenzothiazole or 6-amino-2-cyanobenzothiazole, a functionalized D-luciferin or a functionalized amino-D-luciferin molecule is obtained which is not readily reactive towards luciferase;
b) functionalized 6-hydroxy-2-cyanobenzothiazole or functionalized 6-amino-2-cyanobenzothiazole, wherein the functionalization does not prevent the reactivity towards D-cysteine, functionalized or unfunctionalized aminothiols or functionalized or unfunctionalized 2-aminobenzylamines; and wherein, when reacting with a molecule according to a) or when reacting with an unfunctionalized D-cysteine, a functionalized D-luciferin or a functionalized amino-D-luciferin molecule is obtained, which is not readily reactive towards luciferase;
c) functionalized D-cysteine, wherein the functionalization prevents the reactivity towards 6-hydroxy-2-cyanobenzothiazole or 6-amino-2-cyanobenzothiazole;
d) functionalized 6-hydroxy-2-cyanobenzothiazole or functionalized 6-amino-2-cyanobenzothiazole, wherein the functionalization prevents the reactivity towards D-cysteine.

2. Use of a functionalized D-cysteine according to compounds a) or c) of claim 1 or a functionalized 6-hydroxy-2-cyanobenzothiazole or a functionalized 6-amino-2-cyanobenzothiazole according to compounds b) or d) of claim 1 for the synthesis of D-luciferin, amino-D-luciferin, blocked D-luciferin or blocked amino-D-luciferin, respectively.

3. Method of synthesizing blocked D-luciferin or blocked amino-D-luciferin, wherein
a) a functionalized D-cysteine according to a) of claim 1 is mixed and allowed to react with either a functionalized 6-hydroxy-2-cyanobenzothiazole or a functionalized 6-amino-2-cyanobenzothiazole according to b) of claim 1; or with unfunctionalized 6-hydroxy-2-cyanobenzothiazole or 6-amino-2-cyanobenzothiazole;
or, wherein
b) a functionalized 6-hydroxy-2-cyanobenzothiazole or a functionalized 6-amino-2-cyanobenzothiazole according to b) of claim 1 is mixed and allowed to react with either a functionalized D-cysteine according to a) of claim 1; or with unfunctionalized D-cysteine.

4. Method of synthesizing D-luciferin or amino-D-luciferin comprising the steps of
i) providing either a functionalized D-cysteine according to compounds a) or c) of claim 1 or a functionalized 6-hydroxy-2-cyanobenzothiazole or a functionalized 6-amino-2-cyanobenzothiazole according to compounds b) or d) of claim 1;
ii) providing the respective complementary unfunctionalized or functionalized 6-hydroxy-2-cyanobenzothiazole or 6-amino-2-cyanobenzothiazole or unfunctionalized or functionalized D-cysteine;
iii) adding the components provided in steps i) and ii) to a system containing one or more biomolecules capable of cleaving functional groups off functionalized D-cysteine and/or functionalized 6-hydroxy-2-cyanobenzothiazole or functionalized 6-amino-2-cyanobenzothiazole and allowing them to react.

5. Kit of parts for a screening assay for the detection of molecules in vivo or in vitro comprising
- either a functionalized D-cysteine according to compounds a) or c) of claim 1 or a functionalized 6-hydroxy-2-cyanobenzothiazole or a functionalized 6-amino-2-cyanobenzothiazole according to compounds b) or d) of claim 1;
- the respective complementary unfunctionalized or functionalized 6-hydroxy-2-cyanobenzothiazole or 6-amino-2-cyanobenzothiazole or unfunctionalized or functionalized D-cysteine;
- optionally, luciferase;
- optionally, ATP,
- optionally, magnesium cation source and
- instructions for use.

6. Use of a kit of parts for a screening assay according to claim 5 for the detection of molecules.

7. Use of a kit of parts for a screening assay according to claim 5 for the study of cellular uptake of molecules.

8. Use of a kit of parts for a screening assay according to claim 5 for the detection of a hypoxic environment.

9. Method of detecting biomolecules comprising the steps of
i) providing either a functionalized D-cysteine according to compounds a) or c) of claim 1 or a functionalized 6-hydroxy-2-cyanobenzothiazole or a functionalized 6-amino-2-cyanobenzothiazole according to compounds b) or d) of claim 1;
ii) providing the respective complementary unfunctionalized or functionalized D-cysteine or unfunctionalized or functionalized 6-hydroxy-2-cyanobenzothiazole or 6-amino-2-cyanobenzothiazole compound;
iii) providing luciferase;
iv) contacting the components provided in steps i. and ii. and allowing them to react in the presence of one or more biomolecules studied; and
v) detecting and measuring the luminescence signal generated.

10. Kit of parts for a screening assay for the determination of a sequence cleaved by a specific enzyme within a peptide or protein in vivo or in vitro comprising
i) the peptide or protein bearing at the C-terminal end the sequence cleaved by the specific enzyme followed by a D-cysteine residue,
ii) an unfunctionalized 6-hydroxy-2-cyanobenzothiazole or 6-amino-2-cyanobenzothiazole,
iii) optionally, an enzyme capable of cleaving a sequence within the peptide or protein,
iv) optionally, luciferase,
v) optionally, ATP,
vi) optionally, magnesium cation source,
vii) optionally, a means of detecting luminescence and measuring its intensity, and
viii) instructions for use.

11. Use of a kit of parts for a screening assay according to claim 10 for the determination of a sequence within a peptide or protein cleaved by a specific enzyme.

12. Method of determining a sequence within a peptide or protein cleaved by a specific enzyme comprising
i) providing a peptide or protein bearing at the C-terminal end the sequence cleaved by the specific enzyme followed by a D-cysteine residue,
ii) providing an unfunctionalized 6-hydroxy-2-cyanobenzothiazole or 6-amino-2-cyanobenzothiazole;
iii) providing an enzyme capable of cleaving a sequence within the peptide or protein,
iv) mixing the compounds of steps i), ii) and iii) and allowing them to react,
v) providing luciferase, and
vi) detecting and measuring a luminescence signal.

13. Method of determining the D-cysteine concentration in a cell or tissue sample comprising the steps of:
i) lysing a tissue sample and adding a reducing agent to it,
ii) adding a functionalized or unfunctionalized 6-hydroxy-2-cyanobenzothiazole or 6-amino-2-cyanobenzothiazole according to b) or d) of claim 1,
iii) adding luciferase,
iv) allowing the mixture obtained in step iii) to react,
v) detecting and measuring a luminescence signal,
vi) extrapolating from said signal the D-cysteine concentration.
